# EUROPÄISCHE PATENTSCHRIFT

(11) **EP 1 204 630 B1**
(45) Veröffentlichungstag und Bekanntmachung des Hinweises auf die Patenterteilung: **11.10.2006**
(21) Anmeldenummer: 00951500.8
(22) Anmeldetag: 11.08.2000
(51) Int. Cl.: C07C 69/533, A61K 8/00

(54) **VERZWEIGTE, WEITGEHEND UNGESÄTTIGTE ESTERÖLE**
BRANCHED, SUBSTANTIALLY UNSATURATED ESTER OILS
HUILES-ESTERS RAMIFIEES ET LARGEMENT INSATUREES

(30) Priorität: 20.08.1999 DE 19939566
(43) Veröffentlichungstag der Anmeldung: 15.05.2002
(73) Patentinhaber: Cognis IP Management GmbH, 40589 Düsseldorf (DE)
(72) Erfinder: WESTFECHTEL, Alfred, D-40724 Hilden (DE); HÜBNER, Norbert, D-40597 Düsseldorf (DE)
(86) Internationale Anmeldenummer: PCT/EP2000/007848
(87) Internationale Veröffentlichungsnummer: WO 2001/014309

(56) Entgegenhaltungen:
- DE-A- 4 335 781
- DE-C- 4 422 858
- H. MÖHRING: "Produkte der Dimerisierung ungesättigter Fettsäuren VII: Kinetische Untersuchung der Mono- und Dimeren, die bei er Dimerisierung von Ölsäure entstehen" FAT SCI. TECHNOL., Bd. 94, Nr. 2, 1992, Seiten 41-46, XP002154120 in der Anmeldung erwähnt

## Beschreibung

### Gebiet der Erfindung

Die Erfindung befindet sich auf dem Gebiet der kosmetischen Ölkörper und betrifft weitgehend ungesättigte Esteröle, die sich infolge von Verzweigungen in der Kohlenwasserstoffkette gegenüber linearen Homologen durch signifikant verbesserte Eigenschaften auszeichnen, ein Verfahren zu deren Herstellung sowie ihre Verwendung zur Herstellung von oberflächenaktiven Mitteln.

### Stand der Technik

Unter Esterölen versteht der Fachmann kosmetische Ölkomponenten, bei denen Säure- und Alkoholkomponente zusammengenommen mindestens 20 Kohlenstoffatome aufweisen und die bei Raumtemperatur flüssig vorliegen. Die vorteilhaften Eigenschaften dieser Stoffe sind an das Vorhandensein von einer oder mehrerer Doppelbindungen in der Säure- und/oder Alkoholkomponente geknüpft, was gleichzeitig jedoch auch Probleme aufwirft, da die Esteröle leicht der Autoxidation anheimfallen, sich verfärben und unerwünschte chemische Veränderungen (z.B. Bildung von Peroxiden und Hydroperoxiden) erleben.

Es ist deshalb klar, daß im Markt der Wunsch nach Esterölen mit verbesserter Oxidationsstabilität oder geeigneten Ersatzstoffen besteht, welche über mindestens gleichwertige anwendungstechnische Eigenschaften verfügen. Als Alternative für ungesättigte Esteröle haben indes bislang nur mehr oder minder reine Isostearylalkoholester zur Verfügung gestanden. Zu deren Herstellung ist es jedoch erforderlich, zunächst Ölsäure zu dimerisieren, die Fraktion monomerer, verzweigter Fettsäuren abzutrennen, zu härten, einer fraktionierten Kristallisation zu unterwerfen, die dabei anfallende flüssige, isostearinsäurereiche Fraktion abzutrennen, mit Methanol zu verestern und die Ester anschließend zu den Alkoholen zu hydrieren, welche dann abschließend durch Kondensation mit geeigneten Säuren wieder in Ester überführt werden.

Das oben geschilderte Verfahren ist indes durch die zwei Hydrierschritte technisch aufwendig und liefert mit den Isostearylalkoholsulfaten Ersatzstoffe, die die ungesättigten Esteröle nur bedingt ersetzen können. Somit hat die Aufgabe der vorliegenden Aufgabe darin bestanden, ungesättigte Esteröle zur Verfügung zu stellen, die sich durch verbesserte anwendungstechnische Eigenschaften, vorzugsweise eine höhere Oxidationsstabilität auszeichnen.

### Beschreibung der Erfindung

Gegenstand der Erfindung sind verzweigte, weitgehend ungesättigte Esteröle, dadurch erhältlich, daß man
(a) ungesättigte Fettsäuren mit 16 bis 22 Kohlenstoffatomen in an sich bekannter Weise dimerisiert,
(b) die bei der Dimerisierung anfallende Monomerfraktion abtrennt,
(c) die in dieser Fraktion enthaltenen verzweigten, weitgehend ungesättigten Fettsäuren in die entsprechenden Fettsäuremethylester überführt,
(d) die verzweigten, weitgehend ungesättigten Fettsäuremethylester unter Erhalt der Doppelbindungen zu den entsprechenden verzweigten, weitgehend ungesättigten Fettalkoholen hydriert, und diese
(e) in an sich bekannter Weise verestert.

Überraschenderweise wurde gefunden, daß die verzweigten, weitgehend ungesättigten Esteröle gegenüber den linearen Homologen gleicher Kettenlänge und gleicher Iodzahl eine deutlich höhere Autoxidationsstabilität aufweisen. Weitere Vorteile bestehen in einem verbesserten Spreitvermögen und einer leichteren biologischen Abbaubarkeit.

Ein weiterer Gegenstand der Erfindung betrifft ein Verfahren zur Herstellung von verzweigten, weitgehend ungesättigten Esterölen, bei dem man
(a) ungesättigte Fettsäuren mit 16 bis 22 Kohlenstoffatomen in an sich bekannter Weise dimerisiert,
(b) die bei der Dimerisierung anfallende Monomerfraktion abtrennt,
(c) die in dieser Fraktion enthaltenen verzweigten, verzweigten ungesättigten Fettsäuren in die entsprechenden Fettsäuremethylester überführt,
(d) die verzweigten, weitgehend ungesättigten Fettsäuremethylester unter Erhalt der Doppelbindungen zu den entsprechenden verzweigten, weitgehend ungesättigten Fettalkoholen hydriert, und diese
(e) in an sich bekannter Weise verestert.

### Herstellung der Fettalkohole

Die Dimerisierung von Fettsäuren und die Gewinnung von Monomerfettsäuren aus den Dimerisaten ist aus dem Stand der Technik hinreichend bekannt. In diesem Zusammenhang sei beispielsweise auf die Übersichten von A.Behr et al. [Fat Sci.**Technol. 93, 340 (1991)**] sowie H.Möhring et al. **[ibid. 94, 41 (1992) und 94, 241 (1992)]** verwiesen. Die Abfolge der Schritte (a) bis (d) liefert auf Basis von dimerisierten, vorzugsweise einfach ungesättigten C₁₆- bis C₂₂-Fettsäuren, also Ölsäure, Elaidinsäure, Petroselinsäure, Gadoleinsäure und Erucasäure sowie deren Gemischen verzweigte, weitgehend ungesättigte Fettalkohole im Iodzahlbereich von 45 bis 85. Für eine Reihe von Anwendungen ist dies zweifellos völlig ausreichend, werden jedoch Fettstoffe benötigt, die einen höheren Gehalt an ungesättigten Verbindungen aufweisen, empfiehlt es sich, daß man die bei der Dimerisierung anfallende Monomerfraktion zunächst einer fraktionierten Kristallisation unterwirft und die dabei anfallende flüssige Phase gegebenenfalls nach Destillation der Veresterung unterwirft. Die dabei anfallende Fettsäure und deren Methylester stellen eine schon ziemliche reine Isoölsäure bzw. einen Isoölsäuremethylester dar, die eine lodzahl im Bereich 75 bis 95 aufweisen. In jedem Fall ist es ratsam, die Methylester und/oder die Fettalkohole einer Destillation und/oder fraktionierten Kristallisation ("Winterisierung") zu unterwerfen. Die Veresterung der Fettsäuren mit Methanol erfolgt nach den Verfahren des Stands der Technik und dient dazu Methylester zu erzeugen, die sich vergleichsweise leicht hydrieren lassen. Anstelle der Methylester können selbstverständlich auch andere Niedrigalkylester, wie z.B. Ethyl-, Propyl- oder Butylester erzeugt und dann hydriert werden, die Auswahl des Alkohols ist an sich unkritisch und richtet sich ausschließlich nach wirtschaftlichen Kriterien und Verfügbarkeit. Anstelle der Methyl- bzw. Niedrigalkylester ist es grundsätzlich auch möglich, die Fettsäuren direkt zu verestern, allerdings werden für diesen Zweck dann spezielle Katalysatoren benötigt, die mit den Säuren keine Salze bilden; zudem muß das Reaktormaterial korrosionssicher sein. Auch die Hydrierung der ungesättigten Methylester zu den entsprechenden Alkoholen kann in an sich bekannter Weise erfolgen. Entsprechende Verfahren und Katalysatoren, insbesondere solche auf Basis von Kupfer und Zink, sind beispielsweise den folgenden Druckschriften zu entnehmen: **DE 4335781 C1, EP 0602108 B1, US 3193586** und US 3729520 (Henkel); auf den Inhalt dieser Schriften wird ausdrücklich Bezug genommen.

### Veresterung

Für die Veresterung kommen beispielsweise Monocarbonsäuren in Frage, die der Formel (I) folgen,

**R**^{**1**}**CO-OH** **(I)**

in der R¹CO für einen linearen oder verzweigten, aliphatischen oder aromatischen, gesättigten oder ungesättigten Acylrest mit 1 bis 22 Kohlenstoffatomen steht. Typische Beispiele sind niedere aliphatische Carbonsäuren mit 1 bis 5 Kohlenstoffatomen, wie Ameisensäure, Essigsäure, Propionsäure, Buttersäure und Valeriansäure. Ebenfalls in Frage kommen Fettsäuren mit 6 bis 22 Kohlenstoffatomen, wie z.B. Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Reduktion von Aldehyden aus der Roelen'schen Oxosynthese oder der Dimerisierung von ungesättigten Fettsäuren anfallen. Bevorzugt sind technische Fettsäuren mit 12 bis 18 Kohlenstoffatomen wie beispielsweise Kokos-, Palm-, Palmkern- oder Talgfettsäure. Beispiele für aromatische Carbonsäuren sind Benzoesäure und Zimtsäure. Neben den einwertigen Carbonsäuren kommen auch Dicarbonsäuren sowie mehrwertige, gegebenenfalls hydroxysubstituierte Carbonsäuren mit 2 bis 12 Kohlenstoffatomen in Frage, wie beispielsweise Bernsteinsäure, Maleinsäure, Adipinsäure, Phthalsäure, Äpfelsäure, Weinsäure und Citronensäure sowie deren Gemische. Die Veresterung kann in an sich bekannter Weise durchgeführt werden, d.h. in Gegenwart alkalischer oder saurer Katalysatoren, einer Komponente im Überschuß und der kontinuierlichen Entfernung des Reaktionswassers aus dem Gleichgewicht.

### Gewerbliche Anwendbarkeit

Die neuen verzweigten, weitgehend ungesättigten Esteröle zeichnen sich durch besondere Oxidationsstabilität aus und eignen sich daher als Ölkomponenten zur Herstellung von kosmetischen und/oder pharmazeutischen Zubereitungen, in denen sie in Mengen von 1 bis 90, vorzugsweise 5 bis 50 und insbesondere 10 bis 35 Gew.-% enthalten sein können.

### Kosmetische und/oder pharmazeutische Zubereitungen

Die erfindungsgemäßen verzweigten, weitgehend ungesättigten Esteröle können zur Herstellung von kosmetischen und/oder pharmazeutischen Zubereitungen, wie beispielsweise Haarshampoos, Haarlotionen, Schaumbäder, Duschbäder, Cremes, Gele, Lotionen, alkoholische und wäßrig/alkoholische Lösungen, Emulsionen, Wachs/ Fett-Massen, Stiftpräparaten, Pudern oder Salben dienen. Diese Mittel können ferner als weitere Hilfs- und Zusatzstoffe milde Tenside, Ölkörper, Emulgatoren, Überfettungsmittel, Perlglanzwachse, Konsistenzgeber, Verdickungsmittel, Polymere, Siliconverbindungen, Fette, Wachse, Stabilisatoren, biogene Wirkstoffe, Deodorantien, Antitranspirantien, Antischuppenmittel, Filmbildner, Quellmittel, UV-Lichtschutzfaktoren, Antioxidantien, Hydrotrope, Konservierungsmittel, Insektenrepellentien, Selbstbräuner, Solubilisatoren, Parfümöle, Farbstoffe und dergleichen enthalten.

Typische Beispiele für geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine und/oder Proteinfettsäurekondensate, letztere vorzugsweise auf Basis von Weizenproteinen.

Als weitere Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂₋Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, I-sostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von Hydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂₋Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, Guerbetcarbonate, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, Ringöffnungsprodukte von epoxidierten Fettsäureestern mit Polyolen, Siliconöle und/oder aliphatische bzw. naphthenische Kohlenwasserstoffe, wie z.B. wie Squalan, Squalen oder Dialkylcyclohexane in Betracht.

Als Emulgatoren kommen beispielsweise nichtionogene Tenside aus mindestens einer der folgenden Gruppen in Frage:
➢ Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
➢ Alkyl- und/oder Alkenyloligoglykoside mit 8 bis 22 Kohlenstoffatomen im Alk(en)ylrest und deren ethoxylierte Analoga;
➢ Anlagerungsprodukte von 1 bis 15 Mol Ethylenoxid an Ricinusöl und/oder gehärtetes Ricinusöl;
➢ Anlagerungsprodukte von 15 bis 60 Mol Ethylenoxid an Ricinusöl und/oder gehärtetes Ricinsöl;
➢ Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
➢ Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
➢ Mischester aus Pentaerythrit, Fettsäuren, Citronensäure und Fettalkohol gemäß DE 1165574 PS und/oder Mischester von Fettsäuren mit 6 bis 22 Kohlenstoffatomen, Methylglucose und Polyolen, vorzugsweise Glycerin oder Polyglycerin.
➢ Mono-, Di- und Trialkylphosphate sowie Mono-, Di- und/oder Tri-PEG-alkylphosphate und deren Salze;
➢ Wollwachsalkohole;
➢ Polysiloxan-Polyalkyl-Polyether-Copolymere bzw. entsprechende Derivate;
➢ Polyalkylenglycole sowie
➢ Glycerincarbonat.

Die **Anlagerungsprodukte von Ethylenoxid und/oder von Propylenoxid** an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE 2024051 PS** als Rückfettungsmittel für kosmetische Zubereitungen bekannt.

**Alkyl- und/oder Alkenyloligoglycoside**, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Typische Beispiele für geeignete **Partialglyceride** sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

Als **Sorbitanester** kommen Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

Typische Beispiele für geeignete **Polyglycerinester** sind Polyglyceryl-2 Dipolyhydroxystearate (Dehymuls® PGPH), Polyglycerin-3-Diisostearate (Lameform® TGI), Polyglyceryl-4 Isostearate (Isolan® Gl 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische.

Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure. Palmitinsäure, Stearinsäure, Ölsäure, Behensäure und dergleichen.

Weiterhin können als Emulgatoren **zwitterionische Tenside** verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin.

Schließlich kommen auch **Kationtenside** als Emulgatoren in Betracht, wobei solche vom Typ der Esterquats, vorzugsweise methylquaternierte Difettsäuretriethanolaminester-Salze, besonders bevorzugt sind.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.
Als **Perlglanzwachse** kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Als **Konsistenzgeber** kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten.

Geeignete **Verdickungsmittel** sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium hydroxypropyl hydrolyzed collagen (Lamequat®L/Grünau), quaternierte Weizenpolypeptide, Polyethylenimin, kationische Siliconpolymere, wie z.B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, wie z.B. beschrieben in der **FR 2252840 A** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als **anionische, zwitterionische, amphotere und nichtionische Polymere** kommen beispielsweise Vinylacetat/Crotonsäure-Copolymere, Vinylpyrrolidon/Vinylacrylat-Copolymere, Vinylacetat/Butylmaleat/ Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/ Acrylat-Copolymere, Octylacrylamid/Methylmethacrylat/tert.Butylaminoethylmethacrylat/2-Hydroxyproylmethacrylat-Copolymere, Polyvinylpyrrolidon, VinylpyrrolidonNinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage.

Geeignete **Siliconverbindungen** sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlange von 200 bis 300 Dimethylsiloxan-Einheiten und hydrierten Silicaten handelt. Eine detaillierte Übersicht über geeignete flüchtige Silicone findet sich zudem von Todd et al. in **Cosm.Toil. 91, 27 (1976).**

Typische Beispiele für **Fette** sind Glyceride, als **Wachse** kommen u.a. natürliche Wachse, wie z.B. Candelillawachs, Carnaubawachs, Japanwachs, Espartograswachs, Korkwachs, Guarumawachs, Reis-keimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage.

Als **Stabilisatoren** können Metallsalze von Fettsäuren, wie z.B. Magnesium-, Aluminium- und/oder Zinkstearat bzw. -ricinoleat eingesetzt werden.

Unter **biogenen Wirkstoffen** sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen.
Kosmetische **Deodorantien** (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.

Als **keimhemmende Mittel** sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)harnstoff, 2,4,4'-Trichlor-2'-hydroxydiphenylether (Triclosan), 4-Chlor-3,5-dimethylphenol, 2,2'-Methylen-bis(6-brom-4-chlorphenol), 3-Methyl-4-(1-methylethyl)phenol, 2-Benzyl-4-chlorphenol, 3-(4-Chlorphenoxy)-1,2-propandiol, 3-lod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonolaurat (GML), Diglycerinmonocaprinat (DMC), Salicylsäure-N-alkylamide wie z. B. Salicylsäure-n-octylamid oder Salicylsäure-n-decylamid.

Als **Enzyminhibitoren** sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT, Henkel KGaA, Düsseldorf/FRG). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbnonsäuren und deren Ester wie beispielsweise Citronensäure, Äpfelsäure, Weinsäure oder Weinsäurediethylester, sowie Zinkglycinat.

Als **Geruchsabsorber** eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage. wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstofiverbindungen vom Typ der Ester sind z.B. Benzylacetat, p-tert.-Butylcyclohexylacetat, Linalylacetat. Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Syclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

**Antitranspirantien** (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:
➢ adstringierende Wirkstoffe,
➢ Ölkomponenten,
➢ nichtionische Emulgatoren,
➢ Coemulgatoren,
➢ Konsistenzgeber,
➢ Hilfsstoffe wie z. B. Verdicker oder Komplexierungsmittel und/oder
➢ nichtwässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirkonium-tetrachlorohydrat, Aluminium-Zirkonium-pentachlorohydrat und deren Komplexverbindungen z. B. mit Aminosäuren wie Glycin. Daneben können in Antitranspirantien übliche öllösliche und wasserlösliche Hilfsmittel in geringeren Mengen enthalten sein. Solche öllöslichen Hilfsmittel können z.B. sein:
➢ entzündungshemmende, hautschützende oder wohlriechende ätherische Öle,
➢ synthetische hautschützende Wirkstoffe und/oder
➢ öllösliche Parfümöle.

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

Als **Antischuppenmittel** können Climbazol, Octopirox und Zinkpyrethion eingesetzt werden.

Gebräuchliche Filmbildner sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

Als **Quellmittel** für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in **Cosm.Toil. 108, 95 (1993)** entnommen werden.

Unter **UV-Lichtschutzfaktoren** sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
➢ 3-Benzylidencampher bzw. 3-Benzylidennorcampher und dessen Derivate, z.B. 3-(4-Methyl-benzyliden)campher wie in der **EP 0693471 B1** beschrieben;
➢ 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
➢ Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
➢ Ester der Salicylsäure, vorzugsweise Salicylsäure-2-ethylhexylester, Salicylsäure-4-isopropyl-benzylester, Salicylsäurehomomenthylester;
➢ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
➢ Ester der Benzalmalonsäure, vorzugsweise 4-Methoxybenzmalonsäuredi-2-ethylhexylester;
➢ Triazinderivate, wie z.B. 2,4,6-Trianilino-(p-carbo-2'-ethyl-1'-hexyloxy)-1,3,5-triazin und Octyl Triazon, wie in der **EP 0818450 A1** beschrieben oder Dioctyl Butamido Triazone (Uvasorb® HEB);
➢ Propan-1,3-dione, wie z.B. 1-(4-tert.Butylphenyl)-3-(4'methoxyphenyl)propan-1,3-dion;
➢ Ketotricyclo(5.2.1.0)decan-Derivate, wie in der **EP 0694521 B1** beschrieben.

Als wasserlösliche Substanzen kommen in Frage:
➢ 2-Phenylbenzimidazol-5-sulfonsäure und deren Alkali-, Erdalkali-, Ammonium-, Alkylammonium-, Alkanolammonium- und Glucammoniumsalze;
➢ Sulfonsäurederivate von Benzophenonen, vorzugsweise 2-Hydroxy-4-methoxybenzophenon-5-sulfonsäure und ihre Salze;
➢ Sulfonsäurederivate des 3-Benzylidencamphers, wie z.B. 4-(2-Oxo-3-bomylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxy-dibenzoylmethan (Parsol 1789), 1-Phenyl-3-(4'-isopropylphenyl)-propan-1,3-dion sowie Enaminverbindungen, wie beschrieben in der **DE 19712033 A1** (BASF). Die UV-A und UV-B-Filter können selbstverständlich auch in Mischungen eingesetzt werden. Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächen-behandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in **SÖFW-Journal 122, 543 (1996)** zu entnehmen.

Neben den beiden vorgenannten Gruppen primärer Lichtschutzstoffe können auch sekundäre Lichtschutzmittel vom Typ der **Antioxidantien** eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybuty-rophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Zur Verbesserung des Fließverhaltens können ferner **Hydrotrope**, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
➢ Glycerin;
➢ Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
➢ technische Oligoglyceringemische mit einem Eigenkondensationsgrad von 1,5 bis 10 wie etwa technische Diglyceringemische mit einem Diglyceringehalt von 40 bis 50 Gew.-%;
➢ Methyolverbindungen, wie insbesondere Trimethylolethan, Trimethylolpropan, Trimethylolbutan, Pentaerythrit und Dipentaerythrit;
➢ Niedrigalkylglucoside, insbesondere solche mit 1 bis 8 Kohlenstoffen im Alkylrest, wie beispielsweise Methyl- und Butylglucosid;
➢ Zuckeralkohole mit 5 bis 12 Kohlenstoffatomen, wie beispielsweise Sorbit oder Mannit,
➢ Zucker mit 5 bis 12 Kohlenstoffatomen, wie beispielsweise Glucose oder Saccharose;
➢ Aminozucker, wie beispielsweise Glucamin;
➢ Dialkoholamine, wie Diethanolamin oder 2-Amino-1,3-propandiol.

Als **Konservierungsmittel** eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen. Als **Insekten-Repellentien** kommen N,N-Diethyl-m-toluamid, 1,2-Pentandiol oder Ethyl Butylacetylaminopropionate in Frage, als **Selbstbräuner** eignet sich Dihydroxyaceton.

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, *∝-*Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als **Farbstoffe** können die für kosmetische Zwecke geeigneten und zugelassenen Substanzen verwendet werden, wie sie beispielsweise in der Publikation "**Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt - oder Heißprozesse erfolgen; vorzugsweise arbeitet man nach der Phaseninversionstemperatur-Methode.

### Beispiele

**Beispiel 1**. 23 kg Monomerfettsäure Edenor® 935 (Henkel KGaA) wurden mit 20 kg Methanol 2 h bei 240 °C und 100 bar verestert Nach Abtrennen der Wasser/Methanol-Mischung wurde die gleiche Menge Frisch-Methanol zugesetzt und der Vorgang zweimal wiederholt. Der so erhaltene Ester besaß eine Säurezahl von 0,8. Der Methylester wurde in der Festbettfahrweise an einem Zn-Cr-Katalysator unter Erhalt der Doppelbindung hydriert. Hierbei wurden pro Stunde 0,5 Volumeneinheiten Methylester - bezogen auf das Gesamtvolumen der Anlage - durchgesetzt. Nach Abtrieb des Methanols wurde der Rohalkohol destilliert (3% Vorlauf, 90 % Hauptlauf, 6% Rückstand). Der resultierende Alkohol zeigte eine Hydroxylzahl von 192, eine Verseifungszahl von 0,9 und eine lodzahl von 74; der Festpunkt betrug 25,8 °C. 283,3 g technische Ölsäure (Edenor® PK 1805, Henkel KGaA) wurden mit 292 g des zuvor erhaltenen Isooleylalkohols und 0,17 g Zinn-(II)-Oxalat in einer Rührapparatur mit Wasserabscheider unter Stickstoffeinleitung bei Temperaturen von 200 BIS 220 °C verestert. Nach 2 h wurde eine Säurezahl von 10 erreicht. Danach wurde eine weitere Stunde bei 220°C im Wasserstrahlvakuum verestert. Das resultierende Isooleyloleat besaß eine Säurezahl von 2,5.

**Beispiel 2**. Monomerfettsäure wurde durch Kristallisation aus Methanol/Wasser (Emersol-Verfahren) von geradkettigen, gesättigten Fettsäuren weitgehend befreit. Auf diese Weise wurden ca. 20 Gew.% Fettsäure, überwiegend Palmitin- und Stearinsäure, abgetrennt. Die nach Abdestillation des Lösemittels erhaltene flüssige Fettsäuremischung besaß einen Titer von 5 °C und wurde analog Beispiel 1 zunächst in den Methylester überführt und dann zum ungesättigten Fettalkohol hydriert. Dieser zeigte eine Hydroxylzahl von 191, eine Verseifungszahl von 1,7 und eine lodzahl von 87; der Festpunkt betrug 3,8 °C. 283,3 g technische Elaidinsäure wurden mit 292 g des zuvor erhaltenen Isooleylalkohols und 0,17 g Zinn-(II)-Oxalat in einer Rührapparatur mit Wasserabscheider unter Stickstoffeinleitung bei Temperaturen von 200 BIS 220 °C verestert. Nach 2 h wurde eine Säurezahl von 10 erreicht. Danach wurde eine weitere Stunde bei 220°C im Wasserstrahlvakuum verestert. Das resultierende Isooleylelaidinat besaß eine Säurezahl von 2,5.

In der nachfolgenden Tabelle 1 sind eine Reihe von Formulierungsbeispiele angegeben.

**Kosmetische Zubereitungen (Wasser, Konservierungsmittel ad 100 Gew.-%)**

| **Zusammensetzung (INCI)** | **1** | **2** | **3** | **4** | **5** | **6** | **7** | **8** | **9** | **10** |
|---|---|---|---|---|---|---|---|---|---|---|
| **Texapon® NSO** | 15,0 | 15,0 | 10,0 | - | 20,0 | 8,0 | - | - | - | - |
| Sodium Laureth Sulfate | | | | | | | | | | |
| **Texapon® K 14 S** | - | - | - | - | - | - | - | - | 8,0 | 15,0 |
| Sodium Myreth Sulfate | | | | | | | | | | |
| **Ester gemäß Bsp.1** | 5,0 | 5,0 | 2,4 | 1,0 | 5,0 | 3,0 | 1,0 | 1,0 | 3,0 | 8,0 |
| **Texapon® SB 3** | - | - | - | - | - | 7,0 | - | - | - | - |
| Disodium Laureth Suffosuccinate | | | | | | | | | | |
| **Plantacare® 818** | 5,0 | 5,0 | 4,0 | - | - | - | - | - | 6,0 | 4,0 |
| Coco Glucosides | | | | | | | | | | |
| **Plantacare® 2000** | - | - | - | - | 5,0 | 4,0 | - | - | - | - |
| Decyl Glucoside | | | | | | | | | | |
| **Plantacare® PS 10** | - | - | - | 40,0 | - | - | 16,0 | 17,0 | - | - |
| Sodium Laureth Sulfate (and) Coco Glucosides | | | | | | | | | | |
| **Dehyton® PK 45** | 20,0 | 20,0 | - | - | 8,0 | - | - | - | - | 7,0 |
| Cocamidopropyl Betaine | | | | | | | | | | |
| **Eumulgin® B1** | - | - | - | - | 1,0 | - | - | - | - | - |
| Ceteareth-12 | | | | | | | | | | |
| **Eumulgin® B2** | - | - | - | 1,0 | - | - | - | - | - | - |
| Ceteareth-20 | | | | | | | | | | |
| **Lameform® TGI** | - | - | - | 4,0 | - | - | - | - | - | - |
| Polyglyceryl-3 Isostearate | | | | | | | | | | |
| **Dehymuls® PGPH** | - | - | 1,0 | - | - | - | - | - | - | - |
| Polyglyceryl-2 Dipolyhydroxystearate | | | | | | | | | | |
| **Monomuls® 90-L 12** | - | - | - | - - | - | - | - | - | 1,0 | 1,0 |
| Glyceryl Laurate | | | | | | | | | | |
| **Cetiol® HE** | - | 0,2 | - | - | - | - | - | - | - | - |
| PEG-7 Glyceryl Cocoate | | | | | | | | | | |
| **Eutanol®G** | - | - | - | 1,0 | - | - | - | - | - | - - |
| Octyldodecanol | | | | | | | | | | |
| **Ester gemäß Beispiel H1** | 1,0 | 1,0 | 1,0 | 1,0 | 1,0 | 1,0 | 1,0 | 1,0 | 1,0 | 1,0 |
| **Nutrilan® Keratin W** | - | - | - | - | - | - | - | - | 2,0 | 2,0 |
| Hydrolyzed Keratin | | | | | | | | | | |
| **Nutrilan® 1** | 1,0 | - | - | - | - | 2,0 | - | 2,0 | - | - |
| Hydrolyzed Collagen | | | | | | | | | | |
| **Lamesoft® LMG** | - | - | - | - | - | - | - | - | 1,0 | 5,0 |
| Glyceryl Laurate (and) Potassium Cocoyl Hydrolyzed Collagen | | | | | | | | | | |
| **Gluadin® WK** | 1,0 | 1,5 | 4,0 | 1,0 | 3,0 | 1,0 | 2,0 | 2,0 | 2,0 | - |
| Sodium Cocoyl Hydrolyzed Wheat Protein | | | | | | | | | | |
| **Euperlan® PK 3000 AM** | 5.0 | 3,0 | 4,0 | - | - | - | - | 3,0 | 3.0 | - |
| Glycol Distearate (and) Laureth-4 (and) Cocamidopropyl Betaine | | | | | | | | | | |
| **Panthenol** | - | - | 1,0 | - | - | - | - | - | - | - |
| **Arlypon® F** | 2,6 | 1,6 | - | 1,0 | 1,5 | - | - | - | - | - |
| Laureth-2 | | | | | | | | | | |
| **Highcareen® GS** | 1,0 | 1,0 | 1,0 | 1,0 | 1,0 | 1,0 | 1,0 | 1,0 | 1.0 | 1,0 |
| Betaglucan | | | | | | | | | | |
| **Hydagen® CMF** | 1,0 | 1,0 | 1,0 | 1,0 | 1,0 | 1,0 | 1,0 | 1,0 | 1,0 | 1,0 |
| Chitosan | | | | | | | | | | |
| **Sodium Chloride** | - | - | - | - | - | 1,6 | 2,0 | 2,2 | - | 3,0 |
| **Glycerin** (86 Gew.-%ig) | - | 5,0 | - | - | - | - | - | 1,0 | 3,0 | - |

| | | | | | | | | | | |
|---|---|---|---|---|---|---|---|---|---|---|
| (1-4) Duschbad "Two-in-One), (5-10) Shampoo | | | | | | | | | | |

**Tabelle 2**

| **Kosmetische Zubereitungen (Wasser, Konservierungsmittel ad 100 Gew.-%). Fortsetzung 2** | | | | | | | | | | |
|---|---|---|---|---|---|---|---|---|---|---|
| **Zusammensetzung (INCI)** | **11** | **12** | **13** | **14** | **15** | **16** | **17** | **18** | **19** | **20** |
| **Texapon® NSO** | - | 22,0 | 22,0 | - | 20,0 | - | - | - | - | - |
| Sodium Laureth Sulfate | | | | | | | | | | |
| **Plantacare® 818** | - | 10,0 | - | - | 20,0 | - | - | - | - | - |
| Coco Glucosides | | | | | | | | | | |
| **Plantacare® PS 10** | 22,0 | - | 5,0 | 22,0 | - | - | - | - | - | - |
| Sodium Laureth Sulfate (and) Coco Glucosides | | | | | | | | | | |
| **Dehyton® PK 45** | 15,0 | 10,0 | 15,0 | 15,0 | 20,0 | - | - | - | - | - |
| Cocamidopropyl Betaine | | | | | | | | | | |
| **Emulgade® SE** | - | - | - | - | - | 5,0 | 5,0 | 4,0 | - | - |
| Glyceryl Sterate (and) Ceteareth 12/20 (and) Cetearyl Alcohol (and) Cetyl Palmitate | | | | | | | | | | |
| **Lameform® TGI** | - | - | - | - | - | - | - | - | 4,0 | - |
| Polyglyceryl-3 isostearate | | | | | | | | | | |
| **Dehymuls® PGPH** | - | - | - | - | - | - | - | - | - | 4,0 |
| Polyglyceryl-2 Dipolyhydroxystearate | | | | | | | | | | |
| **Monomuls® 90-O 18** | - | - | - | - | - | - | - | - | 2,0 | - |
| Glyceryl Oleate | | | | | | | | | | |
| **Cetiol® HE** | 2,0 | - | - | 2,0 | 5,0 | - | - | - | - | 2,0 |
| PEG-7 Glyceryl Cocoate | | | | | | | | | | |
| **Cetiol® OE** | - | - | - | - | - | - | - | - | 5,0 | 6,0 |
| Dicaprylyl Ether | | | | | | | | | | |
| **Cetiol®PGL** | - | - | - | - | - | - | - | 3,0 | 10,0 | 9,0 |
| Hexyldecanol (and) Hexyldecyl Laurate | | | | | | | | | | |
| **Cetiol® SN** | - | - | - | - | - | 3,0 | 3,0 | - | - | - |
| Cetearyl Isononanoate | | | | | | | | | | |
| **Cetiol® V** | - | - | - | - | - | 3,0 | 3,0 | - | - | - |
| Decyl Oleate | | | | | | | | | | |
| **Myritol® 318** | - | - | - | - | - | - | - | 3,0 | 5,0 | 5,0 |
| Coco Caprylate Caprate | | | | | | | | | | |
| **Ester gemäß Beispiel H2** | 1.0 | 1,0 | 1.0 | 1,0 | 1,0 | 1,0 | 1,0 | 1,0 | 1,0 | 1,0 |
| **Bees Wax** | - | - | - | - | - | - | - | - | 7,0 | 5,0 |
| **Nutrilan® Elastin E20** | - | - | - | - | - | 2,0 | - | - | - | - |
| Hydrolyzed Elasun | | | | | | | | | | |
| **Nutrilan® I-50** | - | - | - | - | 2,0 | - | 2,0 | - | - | - |
| Hydrolyzed Collagen | | | | | | | | | | |
| **Gluadin® AGP** | 0,5 | 0,5 | 0,5 | - | - | - | - | 0,5 | - | - |
| Hydrolyzed Wheat Gluten | | | | | | | | | | |
| **Gluadin® WK** | 2,0 | 2,0 | 2,0 | 2,0 | 5,0 | - | - | - | 0,5 | 0,5 |
| Sodium Cocoyl Hydrolyzed Wheat Protein | | | | | | | | | | |
| **Euperlan® PK 3000 AM** | 5,0 | - | - | 5,0 | - | - | - | - | - | - |
| Glycol Distearate (and) Laureth-4 (and) Cocamidopropyl Betaine | | | | | | | | | | |
| **Highcareen® GS** | 1,0 | 1,0 | 1,0 | 1,0 | 1,0 | 1,0 | 1,0 | 1,0 | 1,0 | 1,0 |
| Betaglucan | | | | | | | | | | |
| **Hydagen® CMF** | 1,0 | 1,0 | 1,0 | 1,0 | 1,0 | 1,0 | 1,0 | 1,0 | 1,0 | 1,0 |
| Chitosan | | | | | | | | | | |
| **Magnesium Sulfate Hepta Hydrate** | - | - | - | - | - | - | - | - | 1,0 | 1,0 |
| **Glycerin** (86 Gew.%ig) | - | - | - | - | - | 3,0 | 3,0 | 5,0 | 5,0 | 3,0 |

| | | | | | | | | | | |
|---|---|---|---|---|---|---|---|---|---|---|
| (11-15) Schaumbad, (16) Softcreme, (17, 18) Feuchtigkeitsemulsion, (19, 20) Nachtcreme | | | | | | | | | | |

## Patentansprüche

1. Verzweigte, weitgehend ungesättigte Esteröle, **dadurch** erhältlich, dass man
(a) ungesättigte Fettsäuren mit 16 bis 22 Kohlenstoffatomen in an sich bekannter Weise dimerisiert,
(b) die bei der Dimerisierung anfallende Monomerfraktion abtrennt,
(c) die in dieser Fraktion enthaltenen verzweigten, weitgehend ungesättigten Fettsäuren in die entsprechenden Fettsäuremethylester überführt,
(d) die verzweigten, weitgehend ungesättigten Fettsäuremethylester unter Erhalt der Doppelbindungen zu den entsprechenden verzweigten, weitgehend ungesättigten Fettalkoholen hydriert, und diese
(e) in an sich bekannter Weise verestert.

2. Verfahren zur Herstellung von verzweigten, weitgehend ungesättigten Esterölen, bei dem man
(a) ungesättigte Fettsäuren mit 16 bis 22 Kohlenstoffatomen in an sich bekannter Weise dimerisiert;
(b) die bei der Dimerisierung anfallende Monomerfraktion abtrennt,
(c) die in dieser Fraktion enthaltenen verzweigten, weitgehend ungesättigten Fettsäuren in die entsprechenden Fettsäuremethylester überführt,
(d) die verzweigten, weitgehend ungesättigten Fettsäuremethylester unter Erhalt der Doppelbindungen zu den entsprechenden verzweigten, weitgehend ungesättigten Fettalkoholen hydriert und diese
(e) in an sich bekannter Weise verestert.

3. Verfahren nach Anspruch 2, **dadurch gekennzeichnet, dass** man die bei der Dimerisierung anfallende Monomerfraktion zunächst einer fraktionierten Kristallisation unterwirft und die dabei anfallende flüssige Phase gegebenenfalls nach Destillation der Veresterung unterwirft.

4. Verfahren nach den Ansprüchen 2 und/oder 3, **dadurch gekennzeichnet, dass** man die Methylester und/oder die Fettalkohole einer Destillation und/oder fraktionierten Kristallisation unterwirft.

5. Verfahren nach mindestens einem der Ansprüche 2 bis 4, **dadurch gekennzeichnet, dass** man die verzweigten, weitgehend ungesättigten Fettalkohole mit Monocarbonsäuren verestert

6. Verfahren nach mindestens einem der Ansprüche 2 bis 5, **dadurch gekennzeichnet, dass** man die verzweigten, weitgehend ungesättigten Fettalkohole mit Dicarbonsäuren verestert.

7. Verfahren nach mindestens einem der Ansprüche 2 bis 6, **dadurch gekennzeichnet, dass** man die verzweigten, weitgehend ungesättigten Fettalkohole mit Hydroxycarbonsäuren verestert.

8. Verwendung der verzweigten, weitgehend ungesättigten Esteröle nach Anspruch 1 zur Herstellung von kosmetischen und/oder pharmazeutischen Zubereitungen.

## Claims

1. Branched, substantially unsaturated ester oils which are obtainable by
(a) dimerizing unsaturated C₁₆₋₂₂ fatty acids in known manner,
(b) removing the monomer fraction accumulating in the dimerization step,
(c) converting the branched, substantially unsaturated fatty acids present in this fraction into the corresponding fatty acid methyl esters,
(d) hydrogenating the branched, substantially unsaturated fatty acid methyl esters with the double bonds intact to form the corresponding branched, substantially unsaturated fatty alcohols and
(e) esterified in known manner.

2. A process for the production of branched, substantially unsaturated ester oils in which
(a) unsaturated C₁₆₋₂₂ fatty acids are dimerized in known manner,
(b) the monomer fraction accumulating in the dimerization step is removed,
(c) the branched, substantially unsaturated fatty acids present in this fraction are converted into the corresponding fatty acid methyl esters,
(d) the branched, substantially unsaturated fatty acid methyl esters are hydrogenated with the double bonds intact to form the corresponding branched, substantially unsaturated fatty alcohols which are then
(e) esterified in known manner.

3. A process as claimed in claim 2, **characterized in that** the monomer fraction accumulating in the dimerization step is first subjected to fractional crystallization and the liquid phase obtained is esterified, optionally after distillation.

4. A process as claimed in claims 2 and/or 3, **characterized in that** the methyl esters and/or the fatty alcohols are subjected to distillation and/or fractional crystallization.

5. A process as claimed in at least one of claims 2 to 4, **characterized in that** the branched, substantially unsaturated fatty alcohols are esterified with monocarboxylic acids.

6. A process as claimed in at least one of claims 2 to 5, **characterized in that** the branched, substantially unsaturated fatty alcohols are esterified with dicarboxylic acids.

7. A process as claimed in at least one of claims 2 to 6, **characterized in that** the branched, substantially unsaturated fatty alcohols are esterified with hydroxycarboxylic acids.

8. The use of the branched, substantially unsaturated ester oils claimed in claim 1 for the production of cosmetic and/or pharmaceutical preparations.

## Revendications

1. Huiles-esters ramifiées, largement insaturées, que l'on peut obtenir :
(a) en dimérisant des acides gras insaturés comportant 16 à 22 atomes de carbone d'une manière connue en elle même,
(b) en séparant la fraction monomère produite lors de la dimérisation,
(c) en transformant les acides gras ramifiés, largement insaturés contenus dans cette fraction en esters méthyliques d'acides gras correspondants,
(d) en hydrogénant les esters méthyliques d'acides gras ramifiés, largement insaturés tout en conservant les doubles liaisons en les alcools gras ramifiés, largement insaturés, correspondants et
(e) en estérifiant ceux-ci d'une manière connue en elle même.

2. Procédé de fabrication d'huiles esters ramifiées, largement insaturées **caractérisé en ce qu'**
(a) on dimérise des acides gras insaturés comportant 16 à 22 atomes de carbone d'une manière connue en elle même,
(b) on sépare la fraction monomère produite lors de la dimérisation,
(c) on transforme les acides gras ramifiés, largement insaturés contenus dans cette fraction en esters méthyliques d'acides gras correspondants,
(d) on hydrogéne les esters méthyliques d'acides gras ramifiés, largement insaturés tout en conservant les doubles liaisons en les alcools gras ramifiés, largement insaturés, correspondants, et
(e) on estérifie ceux-ci d'une manière connue en elle même.

3. Procédé selon la revendication 2,
**caractérisé en ce qu'**
on soumet d'abord la fraction monomère produite lors de la dimérisation à une cristallisation fractionnée et on estérifie la phase liquide alors produite le cas échéant après distillation.

4. Procédé selon les revendications 2 et/ou 3,
**caractérisé en ce qu'**
on soumet les esters méthyliques et/ou les alcools gras à une distillation et/ ou à une cristallisation fractionnée.

5. Procédé selon au moins l'une des revendications 2 à 4,
**caractérisé en ce qu'**
on estérifie les alcools gras ramifiés, largement insaturés avec des acides monocarboxyliques.

6. Procédé selon au moins l'une des revendications 2 à 5,
**caractérisé en ce qu'**
on estérifie les alcools gras ramifiés, largement insaturés avec des acides dicarboxyliques.

7. Procédé selon au moins l'une des revendications 2 à 6,
**caractérisé en ce qu'**
on estérifie les alcools gras ramifiés, largement insaturés, avec des acides hydroxycarboxyliques.

8. Utilisation des huiles-esters ramifiées, largement insaturées selon la revendication 1 pour fabriquer des préparations cosmétiques et/ou pharmaceutiques.
